# FASCICULE DE BREVET EUROPEEN

(11) **EP 1 098 705 B1**
(45) Date de publication et mention de la délivrance du brevet: **24.09.2003**
(21) Numéro de dépôt: 99931347.1
(22) Date de dépôt: 15.07.1999
(51) Int. Cl.: B01J 38/12, C07D 201/08

(54) **PROCEDE DE REGENERATION D'UN CATALYSEUR D'HYDROLYSE CYCLISANTE D'UN AMINONITRILE EN LACTAME ET ULITISATION DU CATALYSEUR REGENERE POUR LA FABRICATION DE LACTAMES**
REGENIERUNGSVERFAHREN EINES KATALYSATORS ZUR CYCLISIERENDEN HYDROLYSE EINES AMINONITRILS IN LACTAM UND VERWENDUNG DES REGENIERTEN KATALYSATORS ZUR LACTAMHERSTELLUNG
METHOD FOR REACTIVATING A CATALYST FOR CYCLIZING HYDROLYSIS OF AN AMINONITRILE INTO A LACTAM AND USE OF THE REGENERATED CATALYST FOR MAKING LACTAMS

(30) Priorité: 22.07.1998 FR 9809528
(43) Date de publication de la demande: 16.05.2001
(73) Titulaire: Rhodia Polyamide Intermediates, 69192 Saint-Fons (FR)
(72) Inventeur: BRUNELLE, Jean-Pierre, F-78290 Croissy-sur-Seine (FR); NEDEZ, Christophe, F-30340 Salindres (FR)
(74) Mandataire: Esson, Jean-Pierre
(86) Numéro de dépôt international: FR9901729
(87) Numéro de publication internationale: WO00004994

(56) Documents cités:
- EP-A- 0 388 070
- EP-A- 0 604 689
- WO-A-96/22974
- DE-A- 2 126 007
- DE-A- 2 641 429
- US-A- 2 357 484
- US-A- 4 628 085
- US-A- 5 646 277
- CHEMICAL ABSTRACTS, vol. 78, no. 22, 4 juin 1973 (1973-06-04) Columbus, Ohio, US; abstract no. 140908, FUJITA Y. ET AL.: "Reactivation of spent titanium dioxide-copper used in caprolactam synthesis" XP002099193 & JP 47 033081 A (TEIJIN LTD.)
- CHEMICAL ABSTRACTS, vol. 79, no. 22, 3 décembre 1973 (1973-12-03) Columbus, Ohio, US; abstract no. 129705, FUJITA Y. ET AL.: "Reactivation of supported copper catalysts" XP002099194 & JP 47 033087 A (TEIJIN LTD.)
- CHEMICAL ABSTRACTS, vol. 82, no. 26, 30 juin 1975 (1975-06-30) Columbus, Ohio, US; abstract no. 175830, FUJITA Y. ET AL.: "Activation of catalysts" XP002099195 & JP 49 043474 A (TEIJIN LTD.)

## Description

La présente invention concerne un procédé de régénération d'un catalyseur d'hydrolyse cyclisante d'un aminonitrile pour la fabrication de lactames.

Elle se rapporte plus particulièrement à la régénération des catalyseurs solides utilisés dans les procédés de production de lactames par hydrolyse cyclisante d'aminonitriles.

En effet, les lactames, tels que l'ε-caprolactame, sont des composés de base pour la fabrication de nombreux produits et plus particulièrement pour la production de polyamides, tels que le PA 6 et ses copolymères.

Parmi les différents procédés connus de synthèse des lactames, un des procédés est l'hydrolyse cyclisante de l'aminonitrile correspondant, plus particulièrement de l'aminonitrile aliphatique non ramifié correspondant, en présence d'eau, et d'un catalyseur.

Les catalyseurs utilisés dans ces procédés peuvent être classés en plusieurs catégories : les catalyseurs solides massiques tels que les oxydes de métaux décrits dans la demande de brevet WO 98/0669, les catalyseurs comprenant une porosité tels que la silice décrite dans le brevet US 4,628,085 ou plus particulièrement les alumines activées décrites dans le brevet US 2,357,484 et les alumines poreuses décrites dans la demande de brevet internationale WO 96/22974, par exemple

Dans les procédés d'hydrolyse cyclisante des aminonitriles en lactames et plus particulièrement de l'aminocapronitrile en ε-caprolactame, le brevet US5646277 précise que la réalisation en phase vapeur de cette réaction est difficile à mettre en oeuvre notamment à une échelle industrielle car l'activité de ceux-ci est instable.

Pour éviter ces problèmes, ce document préconise de réaliser la réaction d'hydrolyse cyclisante en phase liquide en présence ou non d'un solvant.

Les documents cités précédemment décrivent l'utilisation d'une catalyse hétérogène par des catalyseurs solides tels que les alumines, les silices ou des oxydes métalliques pour la réaction d'hydrolyse cyclisante en phase vapeur d'un aminonitrile. La durée de cycle et l'instabilité de ces catalyseurs ne sont pas évoquées dans ces documents car les essais divulgués correspondent uniquement à des durées de réaction de quelques heures.

La Demanderesse s'est aperçue par des essais de longue durée, c'est-à-dire supérieure à 200 heures, que les catalyseurs solides cités précédemment, et notamment certaines alumines présentent une baisse d'activité catalytique plus ou moins rapide.

Pour permettre d'améliorer l'économie du procédé de fabrication des lactames, et plus généralement l'économie des procédés utilisant un catalyseur, la régénération des catalyseurs usés est une solution possible.

Toutefois, les procédés de régénération sont nombreux et souvent pour un même type de catalyseur, différents selon la réaction catalysée. En outre, selon la nature de la réaction catalysée, la régénération du catalyseur peut être possible ou impossible.

En effet, les causes de désactivation des catalyseurs sont multiples et ne sont pas prévisibles. Par ailleurs, il n'est également pas prévisible qu'un traitement du catalyseur pour améliorer certaines de ces propriétés régénère également l'activité catalytique de celui-ci au moins à un niveau acceptable.

Dans le cas d'une réaction d'hydrolyse cyclisante d'un aminonitrile, aucun document ne décrit la possibilité de régénérer les catalyseurs en fin de cycle.

Un des buts de la présente invention est de remédier à ces inconvénients en proposant un procédé de régénération d'un catalyseur d 'hydrolyse cyclisante d'aminonitrile en lactame.

A cet effet, l'invention propose un procédé de régénération d'un catalyseur d'hydrolyse cyclisante d'aminonitrile en lactame, ledit catalyseur étant un solide, et plus généralement un oxyde simple ou mixte, poreux ou non. Ce catalyseur peut être également constitué par un support poreux sur lequel sont adsorbés ou déposés des éléments catalytiquement actifs.

Ce procédé de régénération consiste à traiter le catalyseur usé ou en fin de cycle, à une température comprise entre 300°C et 600°C par une atmosphère oxydante.

Les caractéristiques générales de la réaction d'hydrolyse cyclisante d'aminonitriles sont décrites ci-après.

Par composé aminonitrile, il faut comprendre les composés de formule générale (I) suivante :

N ≡ C - R - NH₂ (I)

dans laquelle :
R représente un radical aliphatique, cycloaliphatique, arylaliphatique substitué ou non comprenant de 3 à 12 atomes de carbone.

A titre d'exemple, on peut citer les aminonitriles aliphatiques, avantageusement les ω-aminonitriles aliphatiques tels que ω-aminovaleronitrile, ω-aminocapronitrile, ω-aminooctanitrile, ω-aminononanitrile, ω-aminodécanitrile, ω-aminodécanonitrile, ω-aminododécanonitrile, méthyl-aminovaléronitrile.

Le composé préféré et le plus important est l'aminocapronitrile qui conduit à l'ε-caprolactame. Ce dernier composé est le monomère du polyamide 6 utilisé pour la fabrication de différents articles tels que pièces moulées, fils, fibres, filaments, câbles ou films.

L'invention s'applique de manière préférentielle aux catalyseurs solides utilisés notamment dans les réactions d'hydrolyse cyclisante en phase vapeur.

Ainsi, la réaction d'hydrolyse cyclisante nécessite la présence d'eau. Le rapport molaire entre l'eau et l'aminonitrile engagés se situe habituellement entre 0,5 et 50 et de préférence entre 1 et 20. La valeur supérieure de ce rapport n'est pas critique pour l'invention, mais des rapports plus élevés n'ont guère d'intérét pour des questions économiques.

La réaction d'hydrolyse cyclisante peut être réalisée en phase liquide ou vapeur.

Ainsi, dans un mode de réalisation, les réactifs aminonitrile et eau sont engagés à l'état liquide sous pression éventuellement en présence d'un solvant comme décrit dans les brevets US5646277, WO95/14665, W096/00722.

L'aminonitrile et l'eau peuvent être engagés sous forme de leurs mélanges à l'état de vapeurs.

Dans ce cas, les réactifs sont maintenus à l'état de vapeur dans le réacteur chargé avec une quantité déterminée de catalyseur..

Le volume libre du réacteur peut être occupé par un solide inerte tel que, par exemple, du quartz, afin de favoriser la vaporisation et la dispersion des réactifs.

On peut sans inconvénient utiliser tout gaz inerte comme vecteur, tel que l'azote, l'hélium ou l'argon.

La température à laquelle est mis en oeuvre le procédé de l'invention doit être suffisante pour que les réactifs soient bien à l'état de vapeurs. Elle se situe généralement entre 200°C et 450°C et de préférence entre 250°C et 400°C.

Le temps de contact entre l'aminonitrile et le catalyseur n'est pas critique. Il peut varier notamment selon l'appareillage utilisé. Ce temps de contact se situe de préférence entre 0,5 à 200 secondes et encore plus préférentiellement entre 1 et 100 secondes.

La pression n'est pas un paramètre critique du procédé. Ainsi on peut opérer sous des pressions de 10⁻³ bar à 200 bar. De préférence, on mettra en oeuvre le procédé sous une pression de 0,1 à 20 bar.

Il n'est pas exclu d'utiliser un solvant inerte dans les conditions réactionnelles, tel que par exemple un alcool, un alcane, un cycloalcane, un hydrocarbure aromatique ou l'un de ces hydrocarbures précédents halogénés, et d'avoir ainsi une phase liquide dans le flux réactionnel.

Ces conditions de mise en oeuvre de la réaction d'hydrolyse cyclisante sont données uniquement à titre indicatif.

En effet, le procédé de régénération de l'invention s'applique également aux catalyseurs utilisés dans des réactions d'hydrolyse cyclisante mises en oeuvre dans des conditions différentes, notamment pour ceux utilisés quand la réaction est réalisée en phase liquide en présence ou non d'un solvant, ou ceux mis en oeuvre dans les procédés en phase vapeur.

Les catalyseurs qui peuvent être régénérés par le procédé de l'invention sont plus particulièrement les oxydes minéraux présentant une porosité élevée et plus avantageusement les oxydes présentant une macro porosité, c'est à dire dont au moins une partie du volume poreux correspond à des pores de diamètre supérieur à 500Å. Le volume poreux correspondant à la macro porosité est avantageusement supérieur à 5 ml/100g. Ces caractéristiques de volume poreux et porosité concernent les catalyseurs neufs et les catalyseurs régénères par le procédé de l'invention. En effet, un des résultats importants du procédé de l'invention est la régénération de la surface spécifique et de la distribution du volume poreux pour récupérer sensiblement les caractéristiques du catalyseur neuf.

Comme oxydes convenables, on peut citer les oxydes simples ou mixtes des éléments suivants : le silicium, le titane, le zirconium, le vanadium, le niobium, le tantale, le tungstène, le molybdène, le fer, les terres rares, l'aluminium

Ainsi, le procédé de l'invention s'applique notamment aux catalyseurs à base d'alumines décrites dans la demande de brevet WO 96/22974, et à de telles alumines comprenant au moins un autre oxyde simple ou mixte d'éléments adsorbé ou supporté par l'alumine.

Parmi les alumines convenables pour la catalyse de la réaction d'hydrolyse cyclisante d'aminonitriles, le procédé de régénération de l'invention s'applique plus particulièrement et avantageusement aux alumines présentant soit une surface spécifique supérieure à 10 m²/g et un volume poreux total supérieur ou égal à 10 ml/100 g, le volume poreux correspondant aux pores de diamètre supérieur à 500Å étant supérieur ou égal à 10 ml/100 g, soit une surface supérieure à 50 m²/g, un volume poreux total supérieur ou égal à 20ml/100g et un volume poreux correspondant aux pores de diamètre supérieur à 70Å supérieur ou égal à 20 ml/100g, ou une surface spécifique supérieure à 50 m²/g, un volume poreux total supérieur ou égal à 15 ml/100g et un volume poreux correspondant aux pores de diamètre supérieur à 200 Å supérieur ou égal à 15ml/100g, de préférence supérieur ou égal à 20ml/100g.

De telles alumines sont décrites dans la demande de brevet WO 96/22974.

Comme indiqué précédemment, ces alumines peuvent également comprendre des oxydes d'éléments déposés ou adsorbés à la surface des pores pour doper l'activité catalytique. Les oxydes métalliques peuvent être notamment des oxydes d'éléments compris dans la liste le silicium, le titane, le zirconium, le vanadium, le niobium, le tantale, le tungstène, le molybdène, le phosphore, le bore, le fer, les alcalins, les alcalino-terreux, les terres rares.

Le procédé de régénération de l'invention peut également s'appliquer à des catalyseurs à base d'oxydes simples ou mixtes métalliques du type catalyseur massique comme ceux décrits dans la demande de brevet W098/06693.

Le procédé de régénération des catalyseurs d'hydrolyse cyclisante d'aminonitriles consiste, dans une caractéristique préférentielle de l'invention à utiliser comme atmosphère oxydante un mélange de gaz contenant au moins 0,2 % en volume d'oxygène. Avantageusement ce mélange est soit un mélange air/gaz inerte ou oxygène/gaz inerte.

Par gaz inerte, il faut comprendre des gaz qui n'ont pas d'action oxydante ou réductrice tel que l'azote, les gaz rares, le gaz carbonique, la vapeur d'eau.

La teneur en oxygène dans le gaz de traitement est avantageusement faible au début du procédé de régénération du catalyseur. Cette concentration en oxygène peut être augmentée progressivement.

Ainsi, la concentration volumique d'oxygène dans le gaz de traitement est comprise de manière préférentielle entre 1% et 10% (bornes incluses), et varie au cours du procédé entre ces deux limites.

La température de traitement des catalyseurs est un critère important du procédé, car un traitement à une température élevée conduit à un catalyseur régénéré mais présentant une faible activité. Ainsi, la température de traitement est comprise selon une caractéristique préférentielle de l'invention entre 370°C et 500°C, et encore plus avantageusement entre 370°C et 450°C.

Selon un autre mode de réalisation de l'invention, le traitement oxydant des catalyseurs est réalisé avantageusement après un prétraitement par de la vapeur d'eau à une température comprise entre 200 et 500°C, de préférence entre 300 et 400°C. La vapeur d'eau peut être utilisée en mélange avec un gaz vecteur tel qu'un gaz inerte comme l'azote ou de l'air dilué dans un gaz inerte.

Le procédé de régénération est réalisé, par exemple, dans le réacteur contenant le catalyseur, par passage d'un gaz oxydant tel que de l'air dilué dans un gaz inerte comme l'azote . Ce réacteur peut être quelconque et peut être avantageusement constitué par les tubes remplis de catalyseur utilisés pour la réaction d'hydrolyse. Le procédé est réalisé avantageusement sous pression atmosphérique.

Selon un mode opératoire préférentiel, la concentration en oxygène est progressivement augmentée, cette augmentation étant contrôlée et asservie à l'exothermie dégagée par l'oxydation des composés à éliminer. Un autre moyen de réaliser le procédé avec un gaz traitant contenant une concentration plus élevée en oxygène consiste à éliminer les calories produites par des moyens de refroidissement pour éviter un frittage du catalyseur poreux.

L'invention concerne également un procédé de fabrication de lactames par hydrolyse cyclisante d'un aminonitrile en présence d'un catalyseur. Le catalyseur est soit un mélange de catalyseur neuf et de catalyseur régénéré selon le procédé de l'invention, soit un catalyseur régénéré selon le procédé de l'invention.

D'autres détails, avantages de l'invention apparaîtront plus clairement au vu des exemples donnés ci-dessous uniquement à titre indicatif.

### Exemple 1

Une alumine de surface spécifique égale à 139 m²/g présente un volume poreux total de 117 ml/100g, avec des volumes poreux représentés par les pores dont le diamètre est supérieur à 70Å et à 500Å respectivement de 116 ml/100g et de 50 ml/100g.

Cette alumine est utilisée dans une réaction d'hydrolyse cyclisante d'un aminocapronitrile selon des conditions opératoires décrites ci-dessous :

Dans un réacteur cylindrique de 40 mm de diamètre et de hauteur égale à 1 m, 166,5g de catalyseur sont chargés et répartis dans le réacteur de la manière suivante :
- dans un premier tronçon de réacteur 66,7g de catalyseur sont mélangés avec 845 g de billes de verre
- 100g de catalyseur pur dans un second tronçon du réacteur

De l'eau et de l'aminocapronitrile sont injectés selon des débits massiques égaux respectivement à 129 g/h et 200 g/h.

Le réacteur est maintenu à une température de 300°C.

Le taux de transformation initial de l'aminocapronitrile est de 99,5 % , la sélectivité initiale en caprolactame étant supérieure à 99 %.

L'essai est arrêté après un fonctionnement de 800 heures.

Le taux de transformation de l'aminocapronitrile est de alors de 96,5%, la sélectivité en caprolactame étant supérieure à 99%.

L'alumine récupérée présente une surface spécifique (80 m²/g) et un volume poreux ( 88,5 ml/100g) diminués.

Cette alumine est soumise à un traitement de régénération selon l'invention. Pour cela, l'alumine est soumise à un flux gazeux d'azote contenant 2% en volume d'oxygène selon un débit de 1,51/min et un temps de contact de 20s. La température est augmentée de 100°C par heure jusqu'à 300°C puis de 10°C par heure jusqu'à la température finale de traitement. Le taux d'oxygène contenu dans l'azote est progressivement augmenté de 2 à 7%. Le catalyseur sous flux gazeux est maintenu pendant 16 heures à la température finale de 460°C.

L'alumine régénérée présente une surface spécifique de 134m²/g et un volume poreux total de 109 ml/100g.

### Exemple 2

Cette alumine régénérée est utilisée dans une réaction d'hydrolyse cyclisante de l'aminocapronitrile dans des conditions identiques à celles décrites dans l'exemple 1.

Le taux de transformation initial de l'aminocapronitrile lors du 2ème cycle est de 99,4%, la sélectivité en caprolactame étant supérieure à 99%.

Après 500 heures de fonctionnement, le taux de transformation de l'aminocapronitrile est de 96,3%, la sélectivité en caprolactame étant toujours supérieure à 99%.

Ces résultats comparables à ceux obtenus avec le catalyseur neuf lors du 1er cycle démontrent l'efficacité du procédé de régénération de l'invention.

## Revendications

1. Procédé de régénération d'un catalyseur d'hydrolyse cyclisante d'aminonitrile en lactame, ledit catalyseur étant un oxyde solide, **caractérisé en ce que** le catalyseur est traité à une température comprise entre 300°C et 600°C sous une atmosphère oxydante.

2. Procédé selon la revendication 1, **caractérisé en ce que** l'atmosphère oxydante comprend au moins 0,2 % en volume d'oxygène.

3. Procédé selon la revendication 1 ou 2, **caractérisé en ce que** l'atmosphère oxydante est un mélange air/gaz inerte, ou oxygène / gaz inerte.

4. Procédé selon la revendication 3, **caractérisé en ce que** le gaz inerte est choisi dans le groupe comprenant l'azote, les gaz rares, le gaz carbonique, la vapeur d'eau.

5. Procédé selon l'une des revendications 1 à 4, **caractérisé en ce que** la concentration en oxygène dans l'atmosphère oxydante est augmentée progressivement pendant le traitement du catalyseur.

6. Procédé selon l'une des revendications 2 à 5, **caractérisé en ce que** la concentration en oxygène dans l'atmosphère oxydante est comprise entre 1% et 10% en volume.

7. Procédé selon l'une des revendications précédentes, **caractérisé en ce que** la température de traitement est comprie entre 370°C et 500°C.,

8. Procédé selon l'une des revendications précédentes, **caractérisé en ce que** le catalyseur est un oxyde simple ou mixte d'éléments sous forme massique.

9. Procédé selon l'une des revendications 1 à 7, **caractérisé en ce que** le catalyseur comprend un oxyde minéral simple ou mixte d'au moins un élément présentant au moins un volume poreux correspondant à des pores de diamètre supérieur à 500Å (50 nm).

10. Procédé selon la revendication 9, **caractérisé en ce que** l'élément ou les éléments sont choisis dans la liste comprenant le silicium, le titane, le zirconium, le vanadium, le niobium, le tantale, le tungstène, le molybdène, le fer, les terres rares, l'aluminium.

11. Procédé selon l'une des revendications 9 ou 10, **caractérisé en ce que** l'oxyde minéral est l'alumine.

12. Procédé selon l'une des revendications 9 à 11, **caractérisé en ce que** le catalyseur neuf présente soi une surface spécifique supérieure à 10 m²/g et un volume poreux total supérieur ou égal à 10 ml/100 g, le volume poreux correspondant aux pores de diamètre supérieur à 500Å étant supérieur ou égal à 10 ml/100 g, soit une surface spécifique supérieure à 50 m²/g, un volume poreux total supérieur ou égal à 20ml/100g et un volume poreux correspondant aux pores de diamètre supérieur à 70Å supérieur ou égal à 20 ml/100g, ou une surface spécifique supérieure à 50 m²/g, un volume poreux total supérieur ou égal à 15 ml/100g et un volume poreux correspondant aux pores de diamètre supérieur à 200 Å supérieur ou égal à 15ml/100g.

13. Procédé selon l'une des revendications 9 à 12, **caractérisé en ce que** l'alumine comprend au moins un oxyde simple ou mixte d'éléments adsorbés ou déposés à la surface des pores, les éléments étant choisis dans le groupe comprenant le silicium, le titane, le zirconium, le vanadium, le niobium, le tantale, le tungstène, le molybdène, le phosphore, le bore, le fer, les alcalins, les alcalino- terreux, les terres rares.

14. Procédé selon l'une des revendications précédentes, **caractérisé en ce que**, préalablement au traitement par une atmosphère oxydante, le catalyseur à régénérer est soumis à un traitement par de la vapeur d'eau.

15. Procédé selon la rev »endication 14, **caractérisé en ce que** lavapeur d'eau est utilisée en mélange avec un gaz vecteur choisi dans le groupe comprenant les gaz inertes ou de l'air dilué par un gaz inerte, à une température comprise entre 200°C et 500°C.

## Patentansprüche

1. Verfahren zur Regenerierung eines Katalysators zur cyclisierenden Hydrolyse eines Aminonitrils zu Lactam, wobei besagter Katalysator ein festes Oxid ist, **dadurch gekennzeichnet, dass** der Katalysator bei einer Temperatur zwischen 300 °C und 600 °C unter einer oxidierenden Atmosphäre behandelt wird.

2. Verfahren gemäß Anspruch 1, **dadurch gekennzeichnet, dass** die oxidierende Atmosphäre wenigstens 0,2 Volumen-% Sauerstoff umfasst.

3. Verfahren gemäß Anspruch 1 oder 2, **dadurch gekennzeichnet, dass** die oxidierende Atmosphäre ein Gemisch Luft / Inertgas oder Sauerstoff / Inertgas ist.

4. Verfahren gemäß Anspruch 3, **dadurch gekennzeichnet, dass** das Inertgas ausgewählt ist aus der Gruppe, die Stickstoff, die Edelgase, Kohlendioxid, Wasserdampf umfasst.

5. Verfahren gemäß einem der Ansprüche 1 bis 4, **dadurch gekennzeichnet, dass** die Konzentration an Sauerstoff in der oxidierenden Atmosphäre während der Behandlung des Katalysators schrittweise erhöht wird.

6. Verfahren gemäß einem der Ansprüche 2 bis 5, **dadurch gekennzeichnet, dass** die Konzentration an Sauerstoff in der oxidierenden Atmosphäre zwischen 1 Vol.-% und 10 Vol.-% liegt

7. Verfahren gemäß einem der vorhergehenden Ansprüche, **dadurch gekennzeichnet, dass** die Behandlungstemperatur zwischen 370 °C und 500 °C liegt.

8. Verfahren gemäß einem der vorhergehenden Ansprüche, **dadurch gekennzeichnet, dass** der Katalysator ein einfaches oder gemischtes Oxid von Elementen in Masseform ist.

9. Verfahren gemäß einem der Ansprüche 1 bis 7, **dadurch gekennzeichnet, dass** der Katalysator ein einfaches oder gemischtes mineralisches Oxid von wenigstens einem Element umfasst, das wenigstens ein Porenvolumen, das Poren mit einem Durchmesser größer als 50 nm (500 Å) entspricht, aufweist

10. Verfahren gemäß Anspruch 9, **dadurch gekennzeichnet, dass** das Element oder die Elemente ausgewählt sind aus der Liste, die Silicium, Titan, Zirkonium, Vanadium, Niob, Tantal, Wolfram, Molybdän, Eisen, die Seltenen Erden, Aluminium umfasst.

11. Verfahren gemäß einem der Ansprüche 9 oder 10, **dadurch gekennzeichnet, dass** das mineralische Oxid Aluminiumoxid ist.

12. Verfahren gemäß einem der Ansprüche 9 bis 11, **dadurch gekennzeichnet, dass** das als neuer Katalysator verwendete Aluminiumoxid entweder eine spezifische Oberfläche größer als 10 m²/g und ein Gesamtporenvolumen größer oder gleich 10 ml/100 g, wobei das Porenvolumen, das den Poren mit einem Durchmesser größer als 50 nm (500 Å) entspricht, größer oder gleich 10 ml/100 g ist, oder eine spezifische Oberfläche größer als 50 m²/g, ein Gesamtporenvolumen größer oder gleich 20 ml/100 g und ein Porenvolumen, das den Poren mit einem Durchmesser größer als 7 nm (70 Å) entspricht, größer oder gleich 20 ml/100 g oder eine spezifische Oberfläche größer als 50 m²/g, ein Gesamtporenvolumen größer oder gleich 15 ml/100 g und ein Porenvolumen, das den Poren mit einem Durchmesser größer als 20 nm (200 Å) entspricht, größer oder gleich 15 ml/100 g aufweist.

13. Verfahren gemäß einem der Ansprüche 9 bis 12, **dadurch gekennzeichnet, dass** das Aluminiumoxid wenigstens ein einfaches oder gemischtes Oxid von Elementen umfasst, die an der Oberfläche der Poren adsorbiert oder abgeschieden sind, wobei die Elemente ausgewählt sind aus der Gruppe, die Silicium, Titan, Zirkonium, Vanadium, Niob, Tantal, Wolfram, Molybdän, Phosphor, Bor, Eisen, die Alkalimetalle, die Erdalkalimetalle, die Seltenen Erden umfasst.

14. Verfahren gemäß einem der vorhergehenden Ansprüche, **dadurch gekennzeichnet, dass** der zu regenerierende Katalysator vor der Behandlung durch eine oxidierende Atmosphäre einer Behandlung mit Wasserdampf unterzogen wird.

15. Verfahren gemäß Anspruch 14, **dadurch gekennzeichnet, dass** der Wasserdampf im Gemisch mit einem Trägergas, das ausgewählt ist aus der Gruppe, die die Inertgase oder mit einem Inertgas verdünnte Luft umfasst, bei einer Temperatur zwischen 200 °C und 500 °C verwendet wird.

## Claims

1. Process for regenerating a catalyst for the cyclizing hydrolysis of an aminonitrile into a lactam, the said catalyst being a solid oxide, **characterized in that** the catalyst is treated at a temperature of between 300°C and 600°C in an oxidizing atmosphere.

2. Process according to Claim 1, **characterized in that** the oxidizing atmosphere contains at least 0.2% oxygen by volume.

3. Process according to Claim 1 or 2, **characterized in that** the oxidizing atmosphere is an air/inert gas or oxygen/inert gas mixture.

4. Process according to Claim 3, **characterized in that** the inert gas is chosen from the group comprising nitrogen, rare gases, carbon dioxide and water vapour.

5. Process according to one of Claims 1 to 4, **characterized in that** the oxygen concentration in the oxidizing atmosphere is gradually increased during the treatment of the catalyst.

6. Process according to one of Claims 2 to 5, **characterized in that** the oxygen concentration in the oxidizing atmosphere is between 1% and 10% by volume.

7. Process according to one of the preceding claims, **characterized in that** the treatment temperature is between 370°C and 500°C.

8. Process according to one of the preceding claims, **characterized in that** the catalyst is a simple or mixed oxide of elements in bulk form.

9. Process according to one of Claims 1 to 7, **characterized in that** the catalyst comprises a simple or mixed mineral oxide of at least one element having at least a pore volume corresponding to pores having a diameter greater than 50 nm (500 Å).

10. Process according to Claim 9, **characterized in that** the element or elements are chosen from the list comprising silicon, titanium, zirconium, vanadium, niobium, tantalum, tungsten, molybdenum, iron, rare earths and aluminium.

11. Process according to either of Claims 9 and 10, **characterized in that** the mineral oxide is alumina.

12. Process according to one of Claims 9 to 11, **characterized in that** the alumina used as fresh catalyst has either a specific surface area greater than 10 m²/g and a total pore volume greater than or equal to 10 ml/100 g, the pore volume corresponding to pores having a diameter greater than 50 nm (500 Å) being greater than or equal to 10 ml/100 g, or a specific surface area greater than 50 m²/g, a total pore volume greater than or equal to 20 ml/100 g and a pore volume corresponding to pores having a diameter greater than 7nm (70 Å) greater than or equal to 20 ml/100 g, or a specific surface area greater than 50 m²/g, a total pore volume greater than or equal to 15 ml/100 g and a pore volume corresponding to pores having a diameter greater than 20 nm (200 Å) greater than or equal to 15 ml/100 g.

13. Process according to one of Claims 9 to 12, **characterized in that** the alumina comprises at least one simple or mixed oxide of elements adsorbed onto or deposited on the surface of the pores, the elements being chosen from the group comprising silicon, titanium, zirconium, vanadium, niobium, tantalum, tungsten, molybdenum, phosphorus, boron, iron, alkali metals, alkaline-earth metals and rare earths.

14. Process according to one of the preceding claims, **characterized in that**, prior to the treatment with an oxidizing atmosphere, the catalyst to be regenerated is subjected to a treatment with water vapour.

15. Process according to Claim 14, **characterized in that** the water vapour is used in a mixture with a carrier gas chosen from the group comprising inert gases or air diluted with an inert gas, at a temperature of between 200°C and 500°C.
